(19) Europäisches Patentamt
European Patent Office
Office européen des brevets

(11) Veröffentlichungsnummer: **0 174 567**
**B1**

(12) **EUROPÄISCHE PATENTSCHRIFT**

(45) Veröffentlichungstag der Patentschrifft: **21.03.90**

(51) Int. Cl. [5]: **C 07 D 405/06,** A 01 N 43/653 // C07D309/06

(21) Anmeldenummer: **85110930.6**

(22) Anmeldetag: **30.08.85**

(54) **Tetrahydropyran-2-yl-alkenyltriazole, diese enthaltende Fungizide, Verfahren zur Herstellung der Triazolverbindungen und Zwischenprodukte für die Herstellung.**

(30) Priorität: **08.09.84 DE 3433035**

(43) Veröffentlichungstag der Anmeldung: **19.03.86 Patentblatt 86/12**

(45) Bekanntmachung des Hinweises auf die Patenterteilung: **21.03.90 Patentblatt 90/12**

(84) Bennante Vertragsstaaten: **AT BE CH DE FR GB IT LI NL SE**

(56) Entgegenhaltungen:
**EP-A-0 044 993**
**EP-A-0 053 311**
**EP-A-0 055 059**
**EP-A-0 062 236**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Rentzea, Costin, Dr.**
**Richard-Kuhn-Strasse 1-3**
**D-6900 Heidelberg (DE)**
Erfinder: **Goetz, Norbert, Dr.**
**Schoefferstrasse 25**
**D-6520 Worms 1 (DE)**
Erfinder: **Ammermann, Eberhard, Dr.**
**Sachsenstrasse 3**
**D-6700 Ludwigshafen (DE)**
Erfinder: **Pommer, Ernst-Heinrich, Dr.**
**Berliner Platz 7**
**D-6703 Limbrugerhof (DE)**

**EP 0 174 567 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die vorliegende Erfindung betrifft neue Tetrahydropyran-2-yl-alkenyltri-azole, Verfahren zu ihrer Herstellung, diese Verbindungen enthaltende fungizide Mittel und ihre Verwendung als Fungizide, sowie Zwischenprodukte zur Herstellung der Wirkstoffe.

Es ist bekannt, daß Triazolderivate wie beispielsweise das 2,2-Dimethyl-4-(1,2,4-triazol-(1)-yl)-5-phenylpentan-3-on (DE-OS-2 638 470) fungizide Wirksamkeit besitzen. Die Wirkung dieser Stoffe ist bei niedrigen Aufwandmengen und Konzentrationen nicht immer ausreichend.

Es ist ferner bekannt, Dioxanyl-triazolyl-propenderivate (EP-A-0 062 236) oder Cycloalkyl-triazolyl-propenolderivate (EP-A-0 044 993) als Fungizide zu verwenden. Ihre fungizide Wirkung ist unbefriedigend.

Es wurde nun gefunden, daß Verbindungen der Formel I

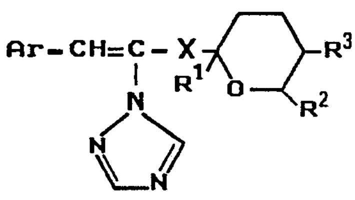

I

in der $R^1$ Methyl bedeutet und $R^2$ und $R^3$ verschieden oder gleich sind und Wasserstoff oder Alkyl mit 1 bis 3 Kohlenstoffatomen bedeuten, Ar Phenyl bedeutet, wobei der Phenylrest durch Alkyl, Alkoxy oder Alkenyl mit jeweils 1 bzw. 2 bis 4 Kohlenstoffatomen, Fluor, Chlor, Brom, Iod, Nitro, Trifluormethyl, Cyan oder Phenoxy substituiert sein kann und

X eine C=O–, –CH(OH)– oder –CH(O–$COR^4$)-Gruppe bedeutet, wobei $R^4$ einen gegebenenfalls durch Halogen oder $C_1$–$C_4$-Alkoxy substituierten $C_1$–$C_3$-Alkylrest bedeutet, sowie deren pflanzenverträgliche Säureadditionssalze ausgezeichnet wirksam gegen Schadpilze sind und sehr gute Pflanzenverträglichkeit zeigen.

Bevorzugt werden Verbindungen mit guter fungizider Wirkung der Formel I, in der Ar einen Phenylrest bedeutet, der durch Fluor, Chlor, Brom, Trifluormethyl, Cyano, Methyl, Ethyl, Propyl, n-Butyl, tert.-Butyl, Methoxy oder durch Ethoxy substituiert sein kann.

Die neuen Verbindungen enthalten Doppelbindungen und gegebenenfalls chirale Zentren. Sie werden im allgemeinen in Form von Z- und E-Isomerengemischen und gegebenenfalls als Racemate oder als Diastereomerengemische erhalten. Die Z- und E-Isomeren lassen sich bei einigen der neuen Verbindungen beispielsweise durch Säulenchromatographie oder aufgrund von Löslichkeitsunterschieden in reiner Form isolieren. Aus einheitlichen Diastereomeren kann man ferner mit bekannten Methoden einheitliche Racemate und Enantiomere erhalten. Alle diese Verbindungen und Gemische werden von der vorliegenden Erfindung ebenfalls umfaßt. Für die Anwendung der neuen Verbindungen als Fungizide sind sowohl die einheitlichen Diastereomeren, Enantiomeren bzw. geometrischen Isomeren Z und E wie auch deren bei der Synthese anfallenden Gemische geeignet. Bevorzugt werden die letzteren verwendet.

$R^2$ und $R^3$ bedeuten beispielsweise Wasserstoff, Methyl, Ethyl und Propyl.

Ar bedeutet beispielsweise Phenyl, einfach bis dreifach substituiertes Phenyl, Halogenphenyl, 4–Fluorphenyl, 2–Chlorphenyl, 3–Chlor– phenyl, 4–Chlorphenyl, 4–Bromphenyl, 2,4–Dichlorphenyl, 3,4–Dichlorphenyl, 3,5–Dichlor–2–methoxyphenyl, 2,3,4–Trichlorphenyl, 2–Methoxyphenyl, 2,4–Dimethoxyphenyl, 3–Methoxyphenyl, 4–Methoxyphenyl, 4–Ethoxyphenyl, 4–tert.–Butoxyphenyl, 4–Methylphenyl, 4–Ethylphenyl, 4–Isopropylphenyl, 4–tert.–Butylphenyl, 3–Nitrophenyl, 4–Nitrophenyl, 3–Trifluormethylphenyl, 4–Trifluormethylphenyl und 4–Phenoxyphenyl.

$R^4$ bedeutet beispielsweise Methyl, Ethyl, n-Propyl, Isopropyl, Chlormethyl, Chlorpropyl und Methoxymethyl.

Pflanzenverträgliche Säureadditionssalze sind z. B. die Salze mit Salzsäure, Schwefelsäure, Phosphorsäure, Dodecylbenzolsulfonsäure.

Die Verbindungen der Formel I lassen sich herstellen, indem man einen Aldehyd der Formel II

$$Ar-CH=O \qquad II,$$

in der Ar die oben genannte Bedeutung hat, mit einem 1-(Tetrahydropyran-2-yl)-2-(1,2,4-triazolyl-(1))-ethan-1-on-Derivat der Formel III

III

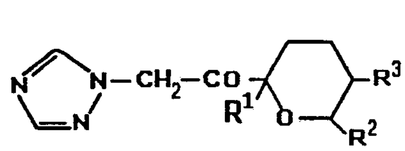

in welcher $R^1$, $R^2$ und $R^3$ die oben angegebenen Bedeutungen haben, kondensiert und die so erhaltenen Verbindungen gegebenenfalls zum Alkohol reduziert und gegebenenfalls danach mit einem Säurehalogenid der Formel IV

$Y - CO - R^4$ IV,

in der $R^4$ die oben genannten Bedeutungen hat und Y Chlor oder Brom bedeutet, oder mit einem Säureanhydrid der Formel V

$(R^4 - CO)_2O$ V,

verestert und die erhaltenen Verbindungen gegebenenfalls in ihre pflanzenverträglichen Säureadditionssalze überführt.

Die Verbindungen II werden mit den Verbindungen III gegebenenfalls in Gegenwart eines Lösungs- oder Verdünnungsmittels und gegebenenfalls unter Zusatz einer anorganischen oder organischen Base und/oder Säure bei Temperaturen zwischen -10 und 150°C gegebenenfalls unter azeotropischer Auskreisung des Reaktionswassers kondensiert. Zu den bevorzugten Lösungs- und Verdünnungsmitteln gehören Wasser, Alkohole wie Methanol, Ethanol, Isopropanol und tert.-Butanol, Kohlenwasserstoffe wie n-Hexan, n-Heptan, n- und iso-Octan, Cyclohexan, Tetrahydronaphthalin, Decahydronaphthalin, Toluol, Xylol, Cumol; Amide wie Dimethylformamid, Dimethylacetamid, N-Methylpyrrolidon und Ether wie Diethylether, Methyl-tert.-butylether, Dioxan, Tetrahydrofuran oder entsprechende Gemische.

Geeignete Basen und Säuren, die gegebenenfalls in katalytischen bis zu stöchiometrischen Mengen verwendet werden können, sind beispielsweise Alkali- und Erdalkalihydroxide, wie Natrium-, Kalium-, Calcium- und Bariumhydroxid, Alkoxide wie Natrium- und Kaliummethoxid, -ethoxid, -isopropoxid und -tert.-butoxid, Acetate wie Natrium- oder Kaliumacetat, Amine wie Pyrrolidin, Piperidin, Triethylamin, N,N-Dimethylcyclohexylamin, ferner anorganische Säuren wie Salzsäure, Phosphorsäure und Schwefelsäure oder organische Säuren wie Ameisensäure, Essigsäure, p-Toluolsulfonsäure oder Trifluormethylsulfonsäure.

Die Umsetzungen werden im allgemeinen bei Temperaturen zwischen 20 und 150°C, drucklos oder unter Druck, kontinuierlich oder diskontinuierlich durchgeführt.

Die Zwischenprodukte der Formel III sind neu und ebenfalls Gegenstand der Erfindung. Sie lassen sich nach an sich bekannten Methoden darstellen. Sie können beispielsweise durch Umsetzung von 1-(Tetrahydropyran-2-yl)-2-halogen-ethan-1-onen der Formel VI

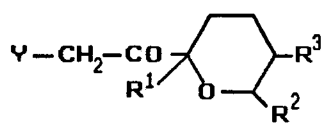

VI

in welcher $R^1$, $R^2$ und $R^3$ die oben angegebenen Bedeutungen haben und Y Chlor oder Brom bedeutet, mit dem Natrium- oder Kaliumsalz des 1,2,4-Triazols erhalten werden.

Die Verbindungen der Formel VI sind ebenfalls neu. Man erhält sie z. B. durch Umsetzung von bekannten 1-(Tetrahydropyran-2-yl)-ethan-1-onen der Formel VII

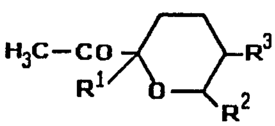

VII

in welcher $R^1$, $R^2$ und $R^3$ die oben angegebenen Bedeutungen haben mit Sulfurylchlorid nach D.P. Wyman und P.R. Kaufman, J.Org.Chem. <u>29</u>, 1956 (1964) oder mit Brom in Fornamid nach H. Bredereck und Mitarb., Chem.Ber. <u>93</u>, 2083 (1960).

Die Umsetzung der Halogenketone der Formel VI mit Triazol oder mit Natrium- oder Kalium-1,2,4-Triazolid erfolgt gegebenenfalls in Gegenwart eines Lösungs- oder Verdünnungsmittels und gegebenenfalls unter Zusatz eines säurebindenden Mittels. Hierzu eignen sich folgende Lösungsmittel: Diethylether, Tetrahydrofuran, Dioxan, Methylenchlorid, Toluol, Xylol, Aceton, Methylethylketon, Dimethylformamid oder N-Methylpyrrolidon.

**Vorschrift 1**

Herstellung des Zwischenproduktes

Zu einer Lösung von 71 g (0,5 Mol) 1-(Tetrahydropyran-2-methyl-2-yl)-ethan-1-on und 45 g (0,53 Mol) Pyrrolidon-(2) in 500 ml Tetrahydrofuran werden bei 50°C in 2 Stunden 272 g (0,55 Mol) Pyrrolidon-Brom-Komplex portionsweise zugegeben. Nach 12 stündigem Rühren bei 50°C wird der weiße Niederschlag von Pyrrolidon-Hydrobromid abgesaugt, mit 50 ml Tetrahydrofuran gewaschen und das Filtrat im Vakuum eingeengt. Man erhält 108 g (98 %) rohes öliges 1-(Tetrahydropyran-2-methyl-2-yl)-2-bromethan-1-on.

Zu einer unter reinem Stickstoff gerührten Suspension von 50 g (0,55 Mol) Natrium-1,2,4-triazolid in 150 ml trockenem Tetrahydrofuran wird eine Lösung von 108 g (0,49 Mol) 1-(Tetrahydropyran-2-methyl-2-yl)-2-bromethan-1-on in 100 ml Tetrahydrofuran bei 25°C in 1 Stunde getropft. Nach 8 stündigem Rückfluß wird der anorganische Niederschlag abfiltriert und das Filtrat eingeengt. Das Rohprodukt wird im Vakuum fraktioniert. Man erhält 95 g (91 % d.Th.) 1-(Tetrahydropyran-2-methyl-2-yl)-2-(1,2,4-triazolyl-(1))-ethan-1-on vom Sdp. 154 – 156°C/0,35 mbar.

Auf entsprechende Weise lassen sich die folgenden Zwischenprodukte darstellen:

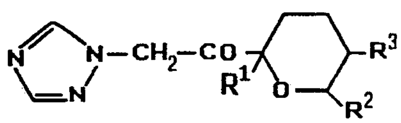

| $R^1$ | $R^2$ | $R^3$ | Phys. Daten |
|---|---|---|---|
| $CH_3$- | $CH_3$- | H | Sdp. 170 – 175°C/0,3 mbar |
| $CH_3$- | H | $CH_3$- | Sdp. 160 – 165°C/0,25 mbar |

**Beispiel 1**

Unter Wasserabscheidung und Kochen am Rückfluß wird eine Mischung aus 31,4 g (0,15 Mol) 1-(Tetrahydropyran-2-methyl-2-yl)-2-(1,2,4-triazolyl-(1)-ethan-1-on, 26,3 g (0,15 Mol) 4-Trifluormethylbenzaldehyd, 2 g Piperidin, 1 g Essigsäure und 300 ml Toluol 18 Stunden gerührt. Das Gemisch wird abgekühlt, dreimal mit je 100 ml Wasser gewaschen, über Natriumsulfat getrocknet und im Vakuum eingeengt. Der Rückstand wird anschließend in 300 ml Diethylether gelöst und bei +5°C mit Chlorwasserstoff begast. Der ausgefallene weiße Niederschlag wird abgesaugt, mit Ether gewaschen und mit 200 ml Methylenchlorid und 200 ml einer 10 %-igen wäßrigen Ammoniaklösung 10 min gerührt. Die organische Schicht wird abgetrennt, über Natriumsulfat getrocknet und eingeengt. Nach dreistündigem Trocknen bei 70°C und 0,1 mbar erhält man 33,1 g (66 % d.Th.) 1-(Tetrahydropyran-2-methyl-2-yl)-2-(1,2,4-triazol-(1))-3-(4-trifluormethylphenyl)-propen-1-on als schwach gelbes Harz (Verbindung Nr. 1).

**Beispiel 2**

Zu einer Lösung von 13,3 g (0,036 Mol) 1-(Tetrahydropyran-2-methyl-2-yl)-2-(1,2,4-triazolyl-(1))-3-(4-trifluormethylphenyl)-propen-1-on in 150 ml Methanol werden zwischen 0°C und 5°C 2,3 g (0,06 Mol) Natriumborhydrid portionsweise zugegeben. Nach 14 stündigem Rühren bei 25°C wird das Gemisch im Vakuum eingeengt. Der Rückstand wird in Methylenchlorid gelöst, dreimal mit Wasser gewaschen, über $Na_2SO_4$ getrocknet und eingeengt. Man erhält 10,9 g (82,5 % d.h.) 1-(Tetrahydropyran-2-methyl-2-yl)-2-(1,2,4-triazolyl-(1))-3-(4-trifluormethylphenyl)-propen-1-ol als schwach gelbes Harz (Verbindung Nr. 2).

IR (Film): 2938, 2867, 1504, 1439, 1325, 1164, 1124, 1068, 1018 $cm^{-1}$.

Entsprechend können durch Wahl der Ausgangsstoffe und entsprechende Anpassung der Verfahrensbedingungen die nachstehend aufgelisteten Verbindungen erhalten werden.

| Nr. | Ar | x | $R^1$ | $R^2$ | $R^3$ | IR-Daten [$cm^{-1}$] bzw. Fp [°C] |
|---|---|---|---|---|---|---|
| 3 | Phenyl | C=O | $CH_3$ | H | H | 1677, 1502, 1120, 1081, 1048 |
| 4 | Phenyl | CH(OH) | CH3 | H | H | 2936, 1502, 1276, 1082, 698 |
| 5 | Phenyl | $CH(OCOCH_3)$ | $CH_3$ | H | H | Öl |
| 6 | Phenyl | $CH(OCOC_2H_5)$ | $CH_3$ | H | H | Öl |
| 7 | Phenyl | $CH(OCOCH_2C1)$ | $CH_3$ | H | H | Öl |
| 8 | Phenyl | C=O | $CH_3$ | $CH_3$ | H | Harz |
| 9 | Phenyl | CH(OH) | $CH_3$ | $CH_3$ | H | Harz |
| 10 | Phenyl | $CH(OCO–CH_2OCH_3)$ | $CH_3$ | $CH_3$ | H | Öl |
| 11 | Phenyl | C=O | $CH_3$ | H | $CH_3$ | Harz |
| 12 | Phenyl | CH(OH) | $CH_3$ | H | $CH_3$ | Harz |
| 13 | Phenyl | $CH(OCOC_3H_7)$ | $CH_3$ | H | $CH_3$ | Harz |
| 14 | 4-Fluorphenyl | C=O | $CH_3$ | H | H | Harz |
| 15 | 4-Fluorphenyl | CH(OH) | $CH_3$ | H | H | Harz |
| 16 | 2-Chlorphenyl | C=O | $CH_3$ | H | H | Öl |
| 17 | 2-Chlorphenyl | CH(OH) | $CH_3$ | H | H | Öl |
| 18 | 3-Chlorphenyl | C=O | $CH_3$ | H | H | Öl |
| 19 | 3-Chlorphenyl | CH(OH) | $CH_3$ | H | H | Öl |

| | | | | | | |
|---|---|---|---|---|---|---|
| 20 | 4-Chlorphenyl | C= | $CH_3$ | H | H | |
| 21 | 4-Chlorphenyl | CH(OH) | $CH_3$ | H | H | 1492, 1276, 1092, 1015, 820 |
| 22 | 4-Chlorphenyl | $CH(OCOC_2H_5)$ | $CH_3$ | H | H | Öl |
| 23 | 4-Chlorphenyl | C=O | $CH_3$ | $CH_3$ | H | 1679, 1502, 1137, 1073, 675 |
| 24 | 4-Chlorphenyl | CH(OH) | $CH_3$ | $CH_3$ | H | 189–185 Isomer I |
| 25 | 4-Chlorphenyl | CH(OH) | $CH_3$ | $CH_3$ | H | 2932, 1504, 1492, 1092 Isomerengemisch |
| 26 | 4-Chlorphenyl | C=O | $CH_3$ | H | $CH_3$ | 1678, 1502, 1092, 1082, 1003 |
| 27 | 4-Chlorphenyl | CH(OH) | $CH_3$ | H | $CH_3$ | 2951, 1492, 1092, 1081, 1068 |
| 28 | 3,4-Dichlorphenyl | C=O | $CH_3$ | H | H | Öl |
| 29 | 3,4-Dichlorphenyl | CH(OH) | $CH_3$ | H | H | Öl |
| 30 | 3,4-Dichlorphenyl | $CH(OCOCHCl_2)$ | $CH_3$ | H | H | Öl |
| 31 | 2,4-Dichlorphenyl | C=O | $CH_3$ | H | H | Harz |
| 32 | 2,4-Dichlorphenyl | CH(OH) | $CH_3$ | H | H | Harz |
| 33 | 2,4-Dichlorphenyl | $CH(OCOCH_3)$ | $CH_3$ | H | H | Harz |
| 34 | 4-Bromphenyl | C=O | $CH_3$ | H | H | 1677, 1502, 1141, 1074, 740 |
| 35 | 4-Bromphenyl | CH(OH) | $CH_3$ | H | H | 2938, 1488, 1073, 1048, 1010 |
| 36 | 4-Bromphenyl | $CH(OCOC_2H_5)$ | $CH_3$ | H | H | Öl |
| 37 | 4-Methylphenyl | C=O | $CH_3$ | H | H | 1675, 1605, 1501, 1119, 1047 |
| 38 | 4-Methylphenyl | CH(OH) | $CH_3$ | H | H | |
| 39 | 4-Methylphenyl | $CH(OCOC_2H_5)$ | $CH_3$ | H | H | 1743, 1176, 1082, 1019, 811 |
| 40 | 3,4-Dimethylphenyl | C=O | $CH_3$ | H | H | Öl |
| 41 | 3,4-Dimethylphenyl | CH(OH) | $CH_3$ | H | H | Öl |
| 42 | 3,4-Dimethylphenyl | $CH(OCOCH_3)$ | $CH_3$ | H | H | Öl |
| 43 | 4-Isopropylphenyl | C=O | $CH_3$ | H | H | Öl |
| 44 | 4-Isopropylphenyl | CH(OH) | $CH_3$ | H | H | Öl |
| 45 | 4-tert.-Butylphenyl | C=O | $CH_3$ | H | H | Öl |
| 46 | 4-tert.-Butylphenyl | CH(OH) | $CH_3$ | H | H | Öl |
| 47 | 4-tert.-Butylphenyl | $CH(OCOC_2H_5)$ | $CH_3$ | H | H | Öl |
| 48 | 4-tert.-Butylphenyl | C=O | $CH_3$ | $CH_3$ | H | Öl |
| 49 | 4-tert.-Butylphenyl | CH(OH) | $CH_3$ | $CH_3$ | H | Öl |
| 50 | 4-tert.-Butylphenyl | $CH(OCOCH_3)$ | $CH_3$ | $CH_3$ | H | Öl |
| 51 | 4-Methoxyphenyl | C=O | $CH_3$ | H | H | 1672, 1598, 1513, 1262, 1179 |
| 52 | 4-Methoxyphenyl | CH(OH) | $CH_3$ | H | H | 2937, 1513, 1251, 1180, 1032 |
| 53 | 4-Methoxyphenyl | $CH(OCOC_2H_5)$ | $CH_3$ | H | H | |
| 54 | 4-Methoxyphenyl | C=O | $CH_3$ | H | $CH_3$ | Öl |
| 55 | 4-Methoxyphenyl | | $CH_3$ | H | $CH_3$ | Öl |
| 56 | 4-Methoxyphenyl | C=O | $CH_3$ | $CH_3$ | H | Öl |
| 57 | 4-Methoxyphenyl | CH(OH) | $CH_3$ | $CH_3$ | H | Öl |
| 58 | 3,4-Dimethoxyphenyl | C=O | $CH_3$ | $CH_3$ | H | Öl |
| 59 | 3,4-Dimethoxyphenyl | CH(OH) | CH3 | $CH_3$ | H | Öl |
| 60 | 3,4-Dimethoxyphenyl | C=O | $CH_3$ | H | H | Öl |
| 61 | 3,4-Dimethoxyphenyl | CH(OH) | $CH_3$ | H | H | Öl |
| 62 | 4-Ethoxyphenyl | C=O | $CH_3$ | H | H | Öl |
| 63 | 4-Ethoxyphenyl | CH(OH) | $CH_3$ | H | H | Öl |
| 64 | 4-Ethoxyphenyl | $CH(OCOC_2H_5)$ | $CH_3$ | H | H | Öl |
| 65 | 4-Trifluormethylphenyl | C=O | $CH_3$ | H | $CH_3$ | 122–124 |
| 66 | 4-Trifluormethylphenyl | CH(OH) | $CH_3$ | H | $CH_3$ | 16O – 162 Isomer I |
| 67 | 4-Trifluormethylphenyl | CH(OH) | $CH_3$ | H | $CH_3$ | 1324, 1131, 1067, 851, Isomerengemisch |
| 68 | 4-Trifluormethylphenyl | C=O | $CH_3$ | $CH_3$ | H | |
| 69 | 4-Trifluormethylphenyl | CH(OH) | $CH_3$ | $CH_3$ | H | 182–184 Isomer I |
| 70 | 4-Trifluormethylphenyl | CH(OH) | $CH_3$ | $CH_3$ | H | 2935, 1325, 1125, 1068, Isomerengemisch |
| 71 | 4-Trifluormethylphenyl | $CH(OCOCH_3)$ | $CH_3$ | $CH_3$ | H | Öl |
| 72 | 4-Trifluormethylphenyl | $CH(OCOC_2H_5)$ | $CH_3$ | $CH_3$ | H | Öl |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blattund Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse – wie Gurken, Bohnen und Kürbisgewächse –.

Die neuen Verbindungen sind insbesondere geeignet zur Bekämpfung folgender Pflanzenkrankheiten:

Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora musae an Bananen,
Pseudocercosporella herpotrichoides an Weizen, Gerste,
Pyricularia oryzae an Reis,
Hemileia vastatrix an Kaffee,
Alternaria solani an Kartoffeln, Tomaten
Plasmopara viticola an Reben sowie Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Ketone (z. B. Cyclohexanon), Amine (z. B. Ethanolamin, Dimethylformamid) und Wasser; Trägerstoffe wie natürliche Gesteinsmehle, (z. B. Kaoline, Tonerden, Talkum, Kreide) und synthetische Gesteinsmehle (z. B. hochdisperse Kieselsäure, Silikate); Emulgiermittel wie nichtionogene und anionische Emulgatoren (z. B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 3 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 9 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 17 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 8 werden den mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 9 werden mit 97 Gewichtsteilen feinteiligem Kaolin innig vermischt. Man erhält auf diese Weise ein Stäubemittel, das 3 Gew.-% des Wirkstoffs enthält.

VII. 30 Gewichtsteile der Verbindung Nr. 18 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 50 werden mit 10 Teilen Natriumsalz eines Phenolsulfonsäure-harnstoffformaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 2 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehydKondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z. B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
0,0-Diethyl-phthalimidophosphonothioat
5-Amino-1-(bis-(dimethylamino)-phosphinyl)-3-phenyl-1,2,4-triazol
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethylthio-phthalimid

N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid

2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
-N-Cyclohexyl-N-methoxy-2,5-dimethyl-furan-3-carbonsäureamid
2-Methyl-benzoesäure-anilid
2-Iod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol

N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hyroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-gluataramid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid.
2-Cyano-N-(ethylaminocarbonyl)-2-methoximino)-acetamid
1-(2-(2,4-Dichlorphenyl)-pentyl)-1H-1,2,4-triazol
2,4-Difluor-alpha-(1H-1,2,4-triazolyl-1-methyl)-benzhydrylalkohol

Für die folgenden Versuche wurde als Vergleich der bekannte Wirkstoff 2,2-Dimethyl-4-(1,2,4-triazolyl-1)-5-phenyl-pentanon-3 (A) verwendet.

**Versuch 1**

Wirksamkeit gegen Gurkenmehltau

Blätter von in Töpfen gewachsenen Gurkenkeimlingen der Sorte "Chinesische Schlange" werden im Zweiblattstadium mit einer Sporensuspension des Gurkenmehltaus (Erysiphe cichoracearum) besprüht. Nach etwa 20 Stunden werden die Versuchspflanzen mit wäßriger Spritzbrühe, die 80 % Wirkstoff und 20 % Emulgiermittel in der Trockensubstanz enthält, bis zur Tropfnässe besprüht. Nach dem Antrocknen des Spritzbelages werden sie anschließend im Gewächshaus bei Temperaturen zwischen 20 und 22°C und 70 bis 80 % relativer Luftfeuchtigkeit aufgestellt. Zur Beurteilung der Wirksamkeit der neuen Stoffe wird das Ausmaß der Pilzentwicklung nach 21 Tagen ermittelt.

Das Ergebnis des Versuches zeigt, daß bei der Anwendung als 0,025 %-ige Spritzbrühe, beispielsweise die Verbindungen 2, 21, 27, 66, 67 und 70 eine bessere fungizide Wirkung zeigen (z. B. 97 %) als der bekannte Wirkstoff A (z. B. 60 %).

**Versuch 2**

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß bei der Anwendung als 0,05 %-ige Spritzbrühe beispielsweise die Verbindungen 27, 34 und 52 eine bessere fungizide Wirkung zeigen (z. B. 90 %) als der bekannte Wirkstoff A (z. B. 60 %).

**Patentansprüche**

1. Tetrahydropyran-2-yl-alkenyltriazole der Formel I

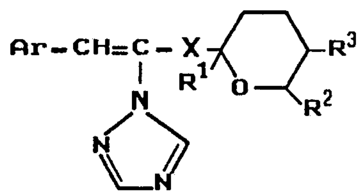

I,

in der $R^1$ Methyl bedeutet und $R^2$ und $R^3$ verschieden oder gleich sind und Wasserstoff oder Alkyl mit 1 bis 3 Kohlenstoffatomen bedeuten,

Ar Phenyl bedeutet, wobei der Phenylrest durch Alkyl, Alkoxy oder Alkenyl mit jeweils 1 bzw. 2 bis 4 Kohlenstoffatomen, Fluor, Chlor, Brom, Iod, Nitro, Trifluormethyl, Cyan oder Phenoxy substituiert sein kann und

X eine C=O–, –CH(OH)– oder –CH(O–COR$^4$)–Gruppe bedeutet, wobei $R^4$ einen gegebenenfalls durch Halogen oder $C_1$–$C_4$–Alkoxy substituierten $C_1$–$C_3$–Alkylrest bedeutet

und ihre pflanzenverträglichen Säureadditionssalze.

2. Verbindungen der Formel I gemäß Anspruch 1, *dadurch gekennzeichnet,* daß Ar Phenyl bedeutet, wobei der Phenylrest durch Fluor, Chlor, Brom, Trifluormethyl, Cyano, Methyl, Ethyl, Propyl, n-Butyl, tert.-Butyl, Methoxy oder durch Ethoxy substituiert sein kann.

3. Verfahren zur Herstellung von Verbindungen gemäß Anspruch 1, *dadurch gekennzeichnet,* daß man einen Aldehyd der Formel

$$Ar - CHO \qquad II,$$

in der Ar die im Anspruch 1 genannte Bedeutung hat, mit einem 1-(Tetrahydropyran-2-yl)-2-(1,2,4-triazolyl-(1))-ethan-1-on Derivat der Formel

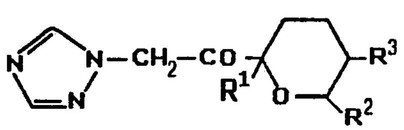

III,

in welcher $R^1$, $R^2$ und $R^3$ die im Anspruch 1 angegebenen Bedeutungen haben, kondensiert und die so erhaltenen Verbindungen gegebenenfalls zum Alkohol reduziert oder zum Alkohol reduziert und gegebenenfalls danach mit einem Säurehalogenid der Formel

$$Y - CO - R^4 \qquad IV,$$

in der $R^4$ die im Anspruch 1 genannte Bedeutung hat, und Y Chlor oder Brom bedeutet, oder mit einem Säureanhydrid der Formel

$$(R^4 - CO)_2O \qquad V,$$

verestert und die erhaltenen Verbindungen gegebenenfalls in ihre pflanzenverträglichen Säureadditionssalze überführt.

4. Verbindungen der Formel III gemäß Anspruch 3 mit den Strukturformeln

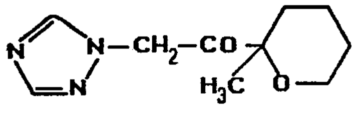

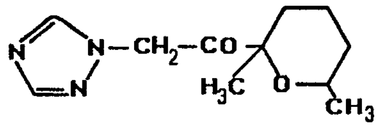

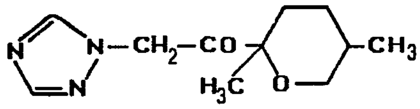

5. Verfahren zur Herstellung von 1-(Tetrahydropyran-2-yl)-2-(1,2,4-triazol-(1))-ethan-1-on-Derivaten der Formel III gemäß Anspruch 4, *dadurch gekenn- zeichnet,* daß man ein Keton der Formel

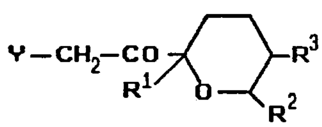

VI,

in der $R_1$ = Methyl; $R_2$, $R_3$ = Wasserstoff oder $R_2$ = Wasserstoff, $R_3$ = Methyl oder $R_2$ = Methyl, $r_3$ = Wasserstoff bedeuten und Y Chlor oder Brom bedeutet, mit dem Natrium -oder Kaliumsalz des 1,2,4Triazols gegebenenfalls in Gegenwart eines Verdünnungsmittels gegebenenfalls in Gegenwart einer Hilfsbase umsetzt.

6. Fungizid, enthältend eine Verbindung gemäß Anspruch 1.

7. Fungizid, enthältend einen festen oder füssigen Trägerstoff und ein Tetrahydropyran-2-yl-alkenyltriazol der Formel I

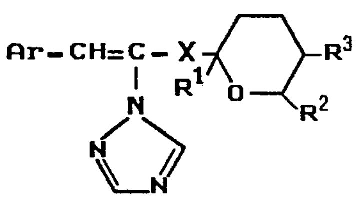

I,

in der $R^1$ Methyl bedeutet und $R^2$ und $R^3$ verschieden oder gleich sind und Wasserstoff oder Alkyl mit 1 bis 3 Kohlenstoffatomen bedeuten,

Ar Phenyl bedeutet, wobei der Phenylrest durch Alkyl, Alkoxy oder Alkenyl mit jeweils 1 bzw. 2 bis 4 Kohlenstoffatomen, Fluor, Chlor, Brom, Iod, Nitro, Trifluormethyl, Cyan oder Phenoxy substituiert sein kann und

X eine C=O–, –CH(OH)– oder –CH(O–COR$^4$)–Gruppe bedeutet, wobei $R^4$ einen gegebenenfalls durch Halogen oder $C_1$–$C_4$–Alkoxy substituierten $C_1$–$C_3$–Alkylrest bedeutet

oder sein pflanzenverträgliches Säureadditionssalz.

8. Verfahren zur Herstellung eines Fungizids, *dadurch gekennzeichnet.* daß man einen festen oder flüssigen Trägerstoff vermischt mit einer Verbindung gemäß Anspruch 1.

9. Verfahren zur Bekämpfung von Pilzen, *dadurch gekennzeichnet,* daß man die Pilze oder die vor Pilzbefall zu schützenden Materialien, Pflanzen, Boden oder Saatgüter behandelt mit einem Tetrahydropyran-2-yl-alkenyltriazol der Formel I

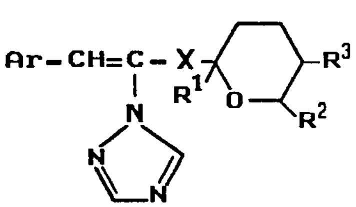

I,

in der $R^1$ Methyl bedeutet und $R^2$ und $R^3$ verschieden oder gleich sind und Wasserstoff oder Alkyl mit 1 bis 3 Kohlenstoffatomen bedeuten,
Ar Phenyl bedeutet, wobei der Phenylrest durch Alkyl, Alkoxy oder Alkenyl mit jeweils 1 bzw. 2 bis 4 Kohlenstoffatomen, Fluor, Chlor Brom, Iod, Nitro, Trifluormethyl, Cyan oder Phenoxy substituiert sein kann und
X eine C=O–, –CH(OH)– oder –CH(O–COR$^4$)-Gruppe bedeutet, wobei $R^4$ einen gegebenenfalls durch Halogen oder $C_1$–$C_4$–Alkoxy substituierten $C_1$–$C_3$–Alkylrest bedeutet
oder sein pflanzenverträgliches Säureadditionssalz.

10. Fungizid gemäß Anspruch 7, *dadurch gekennzeichnet,* daß Ar Phenyl bedeutet, wobei der Phenylrest durch Fluor, Chlor, Brom, Trifluormethyl, Cyano, Methyl, Ethyl, Propyl, n-Butyl, tert.-Butyl, Methoxy oder durch Ethoxy substituiert sein kann.

## Claims

1. A tetrahydropyran-2-ylalkenyltriazole of the formula I

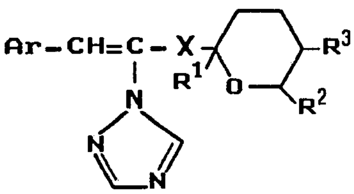

I

where $R^1$ is methyl, $R^2$ and $R^3$ are identical or different and are each hydrogen or alkyl of 1 to 3 carbon atoms, Ar is phenyl which may be substituted by alkyl, alkoxy or alkenyl, each of 1 carbon atom or 2 to 4 carbon atoms, fluorine, chlorine, bromine, iodine, nitro, trifluoromethyl, cyano or phenoxy, and X is C=O–, –CH(OH)– or –CH– (O–COR$^4$), where $R^4$ is $C_1$–$C_3$-alkyl which is unsubstituted or substituted by halogen or $C_1$–$C_4$-alkoxy, and its planttolerated addition salts with acids.

2. A compound of the formula I as claimed in claim 1, wherein Ar is phenyl which may be substituted by fluorine, chlorine, bromine, trifluoromethyl, cyano, methyl, ethyl, propyl, n-butyl, tert-butyl, methoxy or ethoxy.

3. A process for the preparation of a compound as claimed in claim 1, wherein an aldehyde of the formula

Ar – CHO II

where Ar has the meanings stated in claim 1, is condensed with a 1-(tetrahydropyran-2-yl)-2-(1,2,4-triazol-1-yl)ethan-1-one derivative of the formula

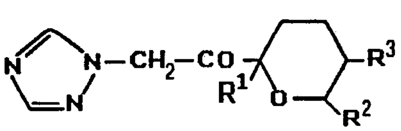

III

where $R^1$, $R^2$ and $R^3$ have the meanings stated in claim 1, and the compound thus obtained is, if required, reduced to the alcohol and, if necessary, then esterified with an acyl halide of the formula

$Y - CO - R^4$ IV

where $R^4$ has the meanings stated in claim 1 and Y is chlorine or bromine, or with an acid anhydride of the formula

$(R^4{-}CO)_2O$ V

and the resulting compound is, if required, converted into its plant-tolerated addition salts with acids.

4. Compounds of the formula III as claimed in claim 3, having the structural formulae

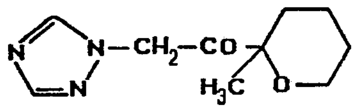

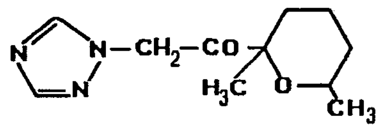

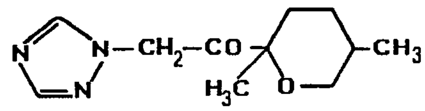

5. A process for the preparation of a 1-(tetrahydro-pyran-2-yl)-2-(1,2,4-triazol-1-yl)-ethan-1-one derivative of the formula

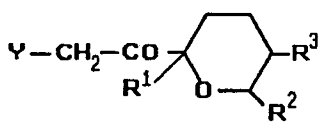

VI

III as claimed in claim IV, wherein a ketone of the formula
where $R^1$ is methyl and $R^2$ and $R^3$ are each hydrogen or $R^2$ is hydrogen and $R^3$ is methyl or $R^2$ is methyl and $R^3$ is hydrogen, and Y is chlorine or bromine, is reacted with the sodium or potassium salt of 1,2,4-triazole in the presence or absence of a diluent and in the presence or absence of an auxiliary base.

6. A fungicide containing a compound as claimed in claim 1.

7. A fungicide containing a solid or liquid carrier and a tetrahydropyran-2-ylalkenyltriazole of the formula

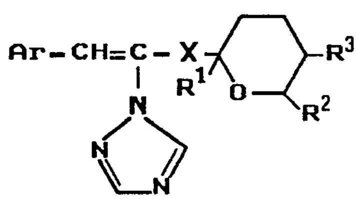

I

where $R^1$ is methyl, $R^2$ and $R^3$ are identical or different and are each hydrogen or alkyl of 1 to 3 carbon atoms, Ar is phenyl which may be substituted by alkyl, alkoxy or alkenyl, each of 1 carbon atom or 2 to 4 carbon atoms, fluorine, chlorine, bromine, iodine, nitro, trifluoromethyl, cyano or phenoxy, and X is C=O–, –CH(OH)– or –CH– (O–$COR^4$), where $R^4$ is $C_1$–$C_3$-alkyl which is unsubstituted or substituted by halogen or $C_1$–$C_4$-alkoxy, and its planttolerated addition salts with acids.

8. A process for the preparation of a fungicide, wherein a solid or liquid carrier is mixed with a compound as claimed in claim 1.

9. A process for controlling fungi, wherein the fungi or the materials, plants, soil or seed to be protected from fungal attack is or are treated with a tetrahydropyran-2-ylalkenyltriazole of the formula I

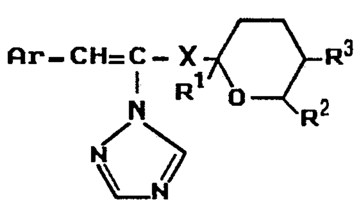

III

where $R^1$ is methyl, $R^2$ and $R^3$ are identical or different and are each hydrogen or alkyl of 1 to 3 carbon atoms, Ar is phenyl which may be substituted by alkyl, alkoxy or alkenyl, each of 1 carbon atom or 2 to 4 carbon atoms, fluorine, chlorine, bromine, iodine, nitro, trifluoromethyl, cyano or phenoxy, and X is C=O–, –CH(OH)– or –CH– (O–$COR^4$), where $R^4$ is $C_1$–$C_3$-alkyl which is unsubstituted or substituted by halogen or $C_1$–$C_4$–alkoxy, and its planttolerated addition salts with acids.

10. A fungicide as claimed in claim 7, wherein Ar is phenyl which may be substituted by fluorine, chlorine, bromine, trifluoromethyl, cyano, methyl, ethyl, propyl, n-butyl, tert-butyl, methoxy or ethoxy.

**Revendications**

1. Tétrahydropyran-2-yl-alcényltriazoles de formule

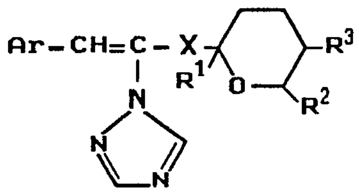

I,

dans laquelle
$R^1$représente méthyle et $R^2$ et $R^3$ sont différents identiques et représentent hydrogène ou alkyle à 3 atomes de carbone,
Ar représente phényle, le reste phényle pouvant être substitué par alkyle, alcoxy ou alcényle ayant chacun 1 ou 2 à 4 atomes de carbone, fluor, chrome, brome, iode, nitro, trifluorométhyle, cyano ou phénoxy à
X représente un groupe C=O, –CH(OH)– ou –CH(O—COR$^4$ $R^4$ représentant un reste alkyle en $C_1$–$C_3$ éventuellement substitué par halogène ou alcoxy en $C_1$–$c_4$ et leurs sels d'addition d'acide acceptables pour le plantes.

2. Composés de formule I, selon la revendication 1, caractérisés par le fait que Ar représente phényle, le reste phényle pouvant être substitué par fluor, chlore, brome, trifluorométhyle, cyano, méthyl, éthyle, propyle, n-butyle, tert-butyle, méthoxy ou par éthoxy.

3. Procédé de préparation de composés selon la revendication 1, caractérisé par le fait que l'on condense une aldéhyde de formule

Ar – CHO II,

dans laquelle Ar a la signification donnée dans la revendication 1, avec un dérivé 1-(tétrahydropyran-2-yl)2-(1,2,4-triazolyl-(1))-éthan-1-one de formule

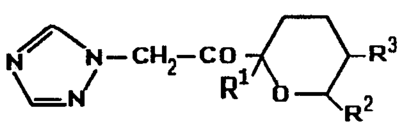

III

dans laquelle $R^1$, $R^2$ et $R^3$ ont les significations données dans la revendiation 1 et on réduit en alcool éventuellement les composés ainsi obtenus et ensuite éventuellement on les estérifie avec un halogénure d'acide de formule

Y - CO - $R^4$ IV,

dans laquelle $R^4$ a la signification donnée dans la revendication 1 et Y représente chlore ou brome, ou avec un anhydride d'acide de formule

$(R^4–CO)_2O$ V,

et on transforme éventuellement les composés obtenus en leurs sels d'addition d'acide acceptables pour les plantes.

4. Composés de formule III selon la revendication 3, ayant les formules de structure

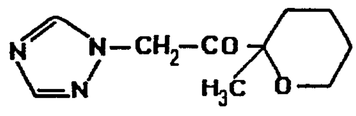

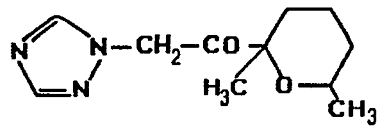

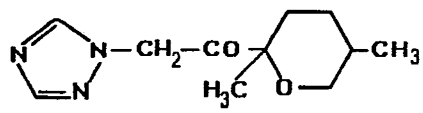

5. Procédé de préparation de dérivés de 1-(tétrahydro-pyran-2-yl)-2-(1,2,4-triazolyl-(1))-éthan-1-one de formule III selon la revendication 4, caractérisé par le fait que l'on fait réagir une cétone de formule

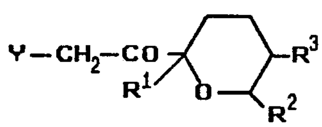

VI

dans laquelle $R_1$ représente méthyle, $R_2$, $R_3$ hydrogène ou $R_2$ hydrogène, $R_3$ méthyle ou $R_2$ = méthyle, $R_3$ = hydrogène, et Y représente chlore ou brome

avec le sel de sodium ou de potassium du 1,2,4-triazole éventuellement en présence d'un diluant, éventuellement en présence d'une base auxiliaire.

6. Fongicide contenant un composé selon la revendication 1.

7. Fongicide contenant un support solide ou liquide et un tétrahydropyran-2-yl-alcényltriazole de formule I

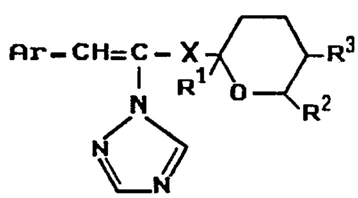

I

dans laquelle

$R^1$ représente méthyle et $R^2$ et $R^3$ sont différents ou identiques et représentent hydrogène ou alkyle à 1 à 3 atomes de carbone,

Ar représente phényle, le reste phényle pouvant être substitué par alkyle, alcoxy ou alcényle ayant chacun 1 ou 2 à 4 atomes de carbone, fluor, chrome, brome, iode, nitro, trifluorométhyle, cyano ou phénoxy et

X représente un groupe C=O, –CH(OH)– ou –CH(O–COR4), $R^4$ représentant un reste alkyle en $C_1$–$C_3$ éventuellement substitué par halogène ou alcoxy en $C_1$–$C_4$ et leurs sels d'addition d'acide acceptables pour les plantes.

8. Procédé de préparation d'un fongicide, caractérisé par le fait que l'on mélange un support solide ou liquide avec un composé selon la revendication 1.

9. Procédé pour lutter contre les champignons, caractérisé par le fait que l'on traite les champignons ou les matériaux, plantes, sols ou semences à protéger de l'attendu de champignons, avec un tétrahydropyran-2-yl-alcényl-triazole de formule I

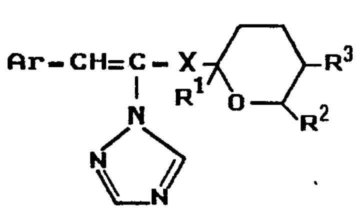

I

dans laquelle

$R^1$ représente méthyle et $R^2$ et $R^3$ sont différents ou identiques et représentent hydrogène ou alkyle à la 3 atomes de carbone,

Ar représente phényle, le reste phényle pouvant être substitué par alkyle, alcoxy ou alcényle ayant chacun 1 ou 2 à 4 atomes de carbone, fluor, chrome, brome iode, nitro, trifluorométhyle, cyano ou phénoxy en

X représente un groupe C=O, –CH(OH)– ou –CH(O–COR$^4$) $R^4$ représentant un reste alkyle en $C_1$–$C_3$ éventuellement substitué par halogène ou alcoxy en $C_1$–$C_4$

et leurs sels d'addition d'acide acceptables pour les plantes.

10. Fongicide selon la revendication 7, caractérisé par le fait que Ar représente phényle, le reste phényle pouvant être substitué par fluor, chlore, brome, trifluorométhyle cyano, méthyle, éthyle, propyle, n-butyle, tert-butyle, méthoxy ou par éthoxy.